# EUROPÄISCHE PATENTSCHRIFT

(11) **EP 4 342 430 B1**
(45) Veröffentlichungstag und Bekanntmachung des Hinweises auf die Patenterteilung: **23.04.2025**
(21) Anmeldenummer: 22197287.0
(22) Anmeldetag: 23.09.2022
(51) Int. Cl.: A61F 2/30, A61F 2/42

(54) **GIESSFORM FÜR FINGERGELENKSPACER**
CASTING MOULD FOR FINGER JOINT SPACER
MOULE POUR ESPACEUR D'ARTICULATION DE DOIGT

(43) Veröffentlichungstag der Anmeldung: 27.03.2024
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE); Nassut, Roman, 61273 Wehrheim (DE)
(74) Vertreter: Heraeus IP

(56) Entgegenhaltungen:
- EP-A1- 2 532 323
- US-A1- 2012 065 738
- US-A1- 2013 183 398
- US-A1- 2018 339 440
- US-A1- 2019 134 857

## Beschreibung

### GEBIET DER ERFINDUNG

Die vorliegende Erfindung betrifft das Gebiet der Medizintechnik, insbesondere Vorrichtungen zur Herstellung medizinischer Implantate, und die damit herstellbaren medizinischen Implantate selbst. Weiterhin betrifft die Erfindung ein Herstellungsverfahren, in dem die erfindungsgemäßen Vorrichtungen zur Herstellung medizinischer Implantate verwendet werden.

### TECHNISCHER HINTERGRUND

Spacer aus ausgehärtetem Polymethylmethacrylatknochenzement, die als temporäre Platzhalter bei septischen Revisionen Verwendung finden, sind seit langem bekannt. Üblich sind hauptsächlich Kniegelenk-, Hüftgelenk-, Schulter und Ellenbogengelenkspacer. In der klinischen Praxis werden einerseits industriell vorgefertigte und andererseits intraoperativ vom OP-Personal gefertigte Spacer verwendet. Für die größeren Gelenke sind unterschiedlichste kommerziell verfügbare Gießformen zur intraoperativen Herstellung bekannt. Diese sind im Allgemeinen zur einmaligen Verwendung bestimmt.

Solche Spacergießformen sind jedoch bisher nicht im Zusammenhang mit Fingergelenken bekannt.

### KURZE BESCHREIBUNG DER ERFINDUNG

Fingergelenke, einschließlich der Daumengelenke, können insbesondere durch Unfälle und auch durch entzündliche Erkrankungen geschädigt werden, so dass diese durch künstliche Gelenke ersetzt werden müssen. Die künstlichen Fingergelenke können durch Mikroorganismen, insbesondere Bakterien, infiziert werden. Die infizierten künstlichen Fingergelenke müssen dann chirurgisch entfernt werden und nach einem Debridement kann neben einer systemischen antibiotischen Behandlung ein Fingergelenk-Spacer, der mit einem Antibiotikum oder mit mehreren Antibiotika beladen sein kann, zur Beruhigung der Infektion in den dem angrenzenden Weich- und Knochengewebe beitragen. Nach der Beruhigung der Infektion und der Entfernung der Fingergelenk-Spacer können dann künstliche Fingergelenke implantiert werden. Somit kann ein Fingergelenk-Spacer im Zusammenhang mit der Behandlung einer Gelenksinfektion als temporärer Platzhalter für ein später einzusetzendes medizinisches Implantat dienen.

Auch bei Gelenkverletzungen, die unter Verlust der Gelenkfunktion eintreten, kann es zu Infektionen des angrenzenden Knochen- und Weichgewebes führen. Hierbei ist eine Beruhigung der Infektion notwendig, um anschließend künstliche Fingergelenke implantieren zu können. Zur Infektionsberuhigung können nach entsprechenden chirurgischem Debridement Spacer, die bevorzugt einen oder mehrere antibiotische Wirkstoffe enthalten, beitragen. Es wäre wünschenswert, Gießformen für Fingergelenkspacer zu entwickeln, mit denen das OP-Personal in einfachster Weise intraoperativ Fingergelenkspacer unter Verwendung von Polymethylmethacrylatknochenzementteig herstellen kann. Nach der Beruhigung der Infektion und der Entfernung der Fingergelenk-Spacer kann dann die Gelenkfunktion durch Implantation künstlicher Fingergelenke wieder hergestellt werden.

US20130183398A1 beschreibt eine Form zum Formen einer temporären Prothese weist mindestens zwei Formelemente auf, die zumindest teilweise voneinander trennbar sind. Die mindestens zwei Formelemente definieren gemeinsam einen im Wesentlichen geschlossenen Innenhohlraum zur Herstellung der temporären Prothese. Die Form weist eine an den mindestens zwei Formelementen angebrachte Befestigungsstruktur auf, um die mindestens zwei Formelemente während der Bildung der temporären Prothese aneinander zu befestigen. Die Befestigungsstruktur ist per Hand und ohne Verwendung eines Werkzeugs von den mindestens zwei Formelementen entfernbar.

EP 2532323A1 betrifft eine Form zur Herstellung der Tibiakomponente eines Kniegelenkspacers, die aus mindestens drei Formteilen besteht. Eine Hülse bildet den Randbereich der Tibiakomponente aus, ein verschiebbar in der Hülse angeordneten Stempel bildet die femurseitigen Oberfläche der Tibiakomponente aus, und ein Deckel bildet die tibiaseitige Oberfläche der Tibiakomponente aus. Die Form weist Fixiereinrichtungen an der Hülse und am Stempel auf, um den Stempel in unterschiedlichen Positionen in der Hülse zu fixieren.

US20180339440A1 offenbart eine Zementformanordnung zum Bilden eines temporären Implantats zur Verabreichung von Antibiotika an eine infizierte Stelle kann eine erste Form und eine zweite Form umfassen. Die erste Form kann ein offenes Ende und eine Innenwand haben. Die erste Form kann einen Hohlraum zum Formen der Tibiakomponente definieren, der einen Hohlraum zum Formen der Plattform und einen Hohlraum zum Formen des Schafts umfasst. Die zweite Form kann einen Körperabschnitt aufweisen, der so konfiguriert ist, dass er von der Innenwand in Richtung des geschlossenen Endes verschiebbar und fortschreitend in den Hohlraum aufgenommen werden kann, der die Tibiakomponente bildet. Durch fortschreitendes Vorschieben der zweiten Form in den Hohlraum, der die Tibiakomponente bildet, wird Zement in dem Hohlraum, der die Tibiakomponente bildet, gegen den Körperabschnitt und die Innenwand gedrückt, um eine einheitliche Tibiakomponente zu bilden, die einen Tibiaschalenabschnitt aufweist, der durch den Hohlraum, der die Plattform bildet, gebildet wird, und einen Schaftabschnitt, der durch den Hohlraum, der den Schaft bildet, gebildet wird.

Eine Aufgabe der Erfindung ist daher die Entwicklung einer möglichst kostengünstig herstellbaren Gießform, beispielsweise aus Kunststoff, zur Herstellung von Spacern mit Hilfe von Polymethylmethacrylat-Knochenzement. Es ist wünschenswert, die Gießform drucklos in einfacher Weise mit hochviskosem Polymethylmethacrylat-Knochenzement zu befüllen. Die Gießform ermöglicht weiterhin die einfache und reproduzierbare Herstellung von Fingergelenkspacern unterschiedlicher Größe.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist. Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein erster Aspekt der Erfindung gemäß Anspruch 1 betrifft eine Vorrichtung zur Herstellung von Fingergelenkspacern. Die Vorrichtung umfasst ein Unterteil und ein Oberteil. Das Unterteil ist mit dem Oberteil verbindbar. Das Unterteil ist mit dem Oberteil an mindestens einer Stelle dauerhaft verbunden. Das Unterteil umfasst mindestens ein erstes Formnest. Das Oberteil umfasst mindestens ein zweites Formnest. Das erste Formnest und das zweite Formnest sind jeweils durch einen Rand definiert, der das Formnest nach außen abgrenzt. Bevorzugt können das erste Formnest und das zweite Formnest an ihren jeweiligen außen liegenden Rändern miteinander verbunden werden. Das erste Formnest ist eingerichtet, mit dem zweiten Formnest gemeinsam einen Hohlraum zu bilden. Dieser Hohlraum wird bevorzugt durch das oben beschriebene Zusammenfügen des ersten Formnests mit dem zweiten Formnest gebildet. Dieser Hohlraum dient zur Aufnahme eines Knochenzementteigs, aus dem ein Fingergelenkspacer, geformt werden kann. Die Form des Spacers wird durch die Form des Hohlraums definiert. Das erste Formnest umfasst eine Spacerkörperform zum Formen eines Spacerkörpers, und das zweite Formnest umfasst eine Schaftform zum Formen eines Schafts. Bevorzugt sind die Schaftform und die Spacerkörperform eingerichtet, miteinander eine kontinuierliche Einheit zu bilden, wenn das Unterteil und das Oberteil in geeigneter Weise miteinander in Kontakt gebracht werden. Entsprechend kann aus der erfindungsgemäßen Vorrichtung bevorzugt ein Spacer aus Knochenzement geformt werden, der einen Spacerkörper und einen Schaft umfasst, welche miteinander eine kontinuierliche Einheit bilden. Der Spacer ist ein Fingergelenkspacer. Der Spacer kann ein artikulierender Spacer oder ein nicht artikulierender Spacer sein. Ein artikulierender Spacer ist eingerichtet, gemeinsam mit einem weiteren Spacer gemeinsam ein Gelenk zu bilden.

In einer Ausführungsform kann das erste Formnest ebenso wie das zweite Formnest eine Schaftform umfassen, sodass die Vorrichtung zur Herstellung eines Spacers mit zwei Schäften geeignet sein kann. Eine solche Spacer kann beispielsweise ein nicht artikulierender Spacer sein. Ein nicht artikulierender Spacer ist in einer Ausführungsform eingerichtet, zwei verschiedene Knochen starr miteinander zu verbinden.

Mit "Knochenzementteig" wird hierin eine formbare Masse bezeichnet, die zu einem Knochenzement aushärtbar ist. Handelsübliche Knochenzemente werden in vielen Fällen als Kit angeboten, welches eine flüssige und eine feste Komponente enthält. Durch Mischen der flüssigen mit der festen Komponente kann der Anwender einen Knochenzementteig herstellen, der für eine begrenzte Zeit leicht verformbar ist. Bereits kurze Zeit nach dem Mischen wird der Knochenzementteig klebfrei, d. h. er haftet bei leichter Berührung mit einem Handschuh nicht mehr an diesem an (wie definiert in ISO 5833:2002). In diesem Zustand kann ein Knochenzementteig bevorzugt in ein Formnest der erfindungsgemäßen Vorrichtung zur Herstellung von Spacern eingefüllt werden, beispielsweise mithilfe eines Rührspatels oder einer geeigneten Austragsvorrichtung. Handelsübliche Knochenzemente werden häufig auf Basis von Polymethylmethacrylat (PMMA) angeboten. Die erfindungsgemäße Vorrichtung kann bevorzugt mit einem solchen PMMA-Knochenzement verwendet werden. Es ist jedoch grundsätzlich auch möglich, andere Arten von Knochenzementen zu verwenden.

Der Begriff "Formnest", wie hierin verwendet, schließt das hierin beschriebene "erste Formnest" und "zweite Formnest" mit ein.

Das erste Formnest umfasst eine Spacerkörperform zum Formen eines Spacerkörpers aus Knochenzement. Das zweite Formnest umfasst eine Schaftform zum Formen eines Schafts aus Knochenzement. Das erste Formnest und das zweite Formnest sind eingerichtet, miteinander einen Hohlraum zu bilden. Die Größe des Hohlraums ist derart dimensioniert, dass geeignete Spacerkörper aus Knochenzement mit der erfindungsgemäßen Vorrichtung hergestellt werden können, die im Bereich eines menschlichen Fingergelenks implantierbar sind. Das Volumen des Hohlraums entspricht der Summe der Volumina des ersten Formnests und des zweiten Formnests, welche gemeinsam den Hohlraum bilden. Beispielsweise kann der Hohlraum zur Aufnahme von Knochenzementteig ein Volumen von 0,1 bis 5 cm³, bevorzugt 0,3 bis 4,0 cm³, umfassen. Je nach den physiologischen Gegebenheiten eines zu behandelnden Patienten können größere oder kleinere Volumina geeignet sein.

Ein Schaft ist ein Teil eines Spacers, der bevorzugt zum Einführen in ein Knochengewebe eingerichtet ist. Die Schaftform kann bevorzugt die Form eines Rotationskörpers umfassen. Ein Rotationskörper ist ein geometrischer Körper, dessen Oberfläche durch Rotation einer erzeugenden Kurve um eine Rotationsachse gebildet wird. Beispiele für Rotationskörper umfassen einen Kegel oder einen Kegelstumpf. In einer Ausführungsform weist die Schaftform die Form eines Kegelstumpfs auf. Die Schaftform kann auch die Form einer Pyramide oder eines Pyramidenstumpfs umfassen. Eine Pyramide besitzt eine mehreckige Grundfläche (d. h. ein Polygon als Grundfläche), und mehrere dreieckige Seitenflächen, welche sich an einem gemeinsamen Punkt berühren. Ein Pyramidenstumpf kann geometrisch gebildet werden, indem man von einer Pyramide (Ausgangspyramide) parallel zur Grundfläche an den Mantelflächen eine kleinere, ähnliche Pyramide (Ergänzungspyramide) abschneidet.

Die Spacerkörperform kann bevorzugt im Wesentlichen quaderförmig oder im Wesentlichen halbkugelförmig ausgebildet sein. Eine "im Wesentlichen quaderförmige" Spacerkörperform kann auch eine gewölbte Fläche umfassen, die von einer geometrisch exakten quaderförmigen Grundform geringfügig abweicht, wie nachfolgend beschrieben.

Die Spacerkörperform dient dem Formen eines Spacerkörpers, welcher bevorzugt eine artikulierende Fläche umfassen kann. Eine "artikulierende Fläche" ist bevorzugt eingerichtet, mit einer korrespondierenden weiteren artikulierenden Fläche ein Gelenk zu bilden, ähnlich wie bei einem menschlichen Fingergelenk. Beispielsweise kann eine rechteckig-konkave artikulierende Fläche in eine rechteckig-konvexe artikulierende Fläche eingreifen, um ein scharnierartiges Gelenk zu bilden. In vergleichbarer Weise kann eine halbkugelförmige artikulierende Fläche in eine korrespondierende Fläche mit einer halbkugelförmigen Aussparung eingreifen, ähnlich einem Kugelgelenk. Durch die derart geeignete Zusammenwirkung zweier erfindungsgemäßer Spacer wird es bevorzugt ermöglicht, dass ein Patient, dem solche Spacer implantiert werden, weiterhin seine Finger in ähnlicher Weise wie mit einem natürlichen Gelenk bewegen kann. Beispiele für das oben beschriebene Zusammenwirken zweier aufeinander abgestimmter artikulierender Flächen nach Art eines Scharniergelenks bzw. Kugelgelenks sind in den anhängenden Zeichnungen dargestellt.

Dementsprechend umfasst die erfindungsgemäße Vorrichtung bevorzugt eine Spacerkörperform mit einer Stirnfläche, welche eingerichtet ist, eine artikulierende Fläche zu formen, die geeignet ist, mit einer weiteren artikulierenden Fläche eines korrespondierenden Spacerkörpers gemeinsam ein Gelenk zu bilden.

In einigen Ausführungsformen ist es demnach bevorzugt, dass die Stirnfläche der Spacerkörperform eine gewölbte Form aufweist, beispielsweise eine konvexe oder konkave Form. Hierdurch können beispielsweise Spacer abgeformt werden, deren Spacerkörper eine artikulierende Fläche mit einer konkaven Form aufweisen, und mit einem weiteren Spacerkörper mit einer korrespondierenden artikulierenden Fläche mit einer konvexen Form gemeinsam ein Gelenk bilden können.

Des Weiteren umfasst die Vorrichtung ein biegsames Verbindungselement, welches das Unterteil mit dem Oberteil verbindet. Bevorzugt ist das Verbindungselement jeweils randständig an dem Unterteil und dem Oberteil angeordnet. Das Verbindungselement in Form eines Folienscharniers verbindet das Unterteil mit dem Oberteil.

In einer weiteren Ausführungsform umfasst die Vorrichtung ein erstes Befestigungselement und ein dazu komplementäres zweites Befestigungselement. Durch Eingreifen des ersten Befestigungselements in das zweite Befestigungselement kann das Oberteil mit dem Unterteil verbunden werden. Hierzu kann das Oberteil ein erstes Befestigungselement umfassen, und das Unterteil kann ein dazu passendes zweites Befestigungselement umfassen, oder umgekehrt. Beispielsweise kann das Oberteil einen Stift enthalten, welcher in eine Buchse bzw. Aussparung auf dem Unterteil einrasten kann, um das Oberteil fest mit dem Unterteil zu verklemmen. Hierdurch können das erste Formnest und das zweite Formnest in passender Position miteinander verbunden und stabil in dieser Position gehalten werden, um den Hohlraum zur Formung eines Spacers zu bilden. In ähnlicher Weise kann eine Klemme oder Klammer, gegebenenfalls mit einem geeigneten Gegenstück, als Befestigungselement vorgesehen sein.

In einer bevorzugten Ausführungsform umfasst die erfindungsgemäße Vorrichtung sowohl ein Verbindungselement als auch ein erstes Befestigungselement und ein dazu komplementäres zweites Befestigungselement. Hierdurch können Unterteil und Oberteil besonders stabil miteinander verbunden und relativ zueinander in einer gewünschten Orientierung fixiert werden.

In einer weiteren Ausführungsform umfasst die Vorrichtung eine Rille, die umlaufend entlang mindestens eines der Formnester angeordnet ist. Bevorzugt ist die Rille in räumlicher Nähe zu einem Rand eines Formnest angeordnet. Der Rand eines Formnest bezeichnet eine Kante, an welcher die hierin beschriebene Geometrie eines Formnests, die zur Bildung eines Hohlraums mit einem zweiten Formnest eingerichtet ist, nach außen abgegrenzt ist.

Die Vorrichtung kann weiterhin einen Steg umfassen, der in ähnlicher Weise umlaufend entlang eines der Formnester angeordnet ist, sodass er in eine entsprechende gegenüberliegende Rille eines anderen Formnest eingreifen kann. Hierdurch kann das Unterteil fest mit dem Oberteil verbunden werden, um den gemeinsam durch die beiden Formnester gebildeten Hohlraum nach außen abzudichten.

In einer bevorzugten Ausführungsform kann die Rille tiefer sein als die Höhe eines dazu passenden Stegs, so dass durch Eingreifen des Stegs in die Rille ein Leerraum zwischen dem Steg und der Rille ausgebildet werden kann. Dieser Leerraum kann bevorzugt zur Aufnahme überschüssigen Knochenzements eingerichtet sein. Hierdurch kann der Bildung von Graten bei der Abformung von Spacern entgegengewirkt werden, da der von dem Leerraum aufgenommene überschüssige Knochenzement nicht unmittelbar auf der Oberfläche des geformten Spacers verbleibt.

In einer weiteren Ausführungsform können das Unterteil und/oder das Oberteil eine Öffnung zum Entlüften des Hohlraums umfassen. Bevorzugt umfassen sowohl das Unterteil als auch das Oberteil jeweils mindestens eine solche Öffnung. Besonders vorteilhaft kann es sein, sowohl an der Spitze der Schaftform als auch an mehreren Ecken der Spacerkörperform solche Öffnungen vorzusehen. Mithilfe einer solchen Öffnung kann sichergestellt werden, dass die Formnester und der Hohlraum vollständig mit Knochenzementteig gefüllt werden können. Die durch den Knochenzementteig verdrängte Luft kann durch eine Öffnung aus dem Formnest entweichen.

Die erfindungsgemäße Vorrichtung kann grundsätzlich aus jedem geeigneten Material bestehen. Biegsame, elastische Materialien können die Entnahme der abgeformten Spacer erleichtern. Die Vorrichtung kann ein Metall oder Polymer umfassen. Beispiele für geeignete Polymere umfassen Kautschuk, Silikonkautschuk, Synthesekautschuk, Ethylen-Propylen-DienKautschuk (EPDM), Polyethylen, Polyetheretherketon, und Polypropylen. Vorrichtungen aus Polypropylen erlauben eine besonders leichte Entnahme des ausgehärteten Knochenzements aus der Vorrichtung. In einigen Ausführungsformen ist die Vorrichtung aus einem transparenten oder zumindest transluzenten, d. h. optisch durchscheinenden, Material gebildet. Hierbei bieten sich entsprechende transparente oder transluzente Polymere an. Hierdurch kann der Verwender der Vorrichtung kontrollieren, ob die Formnester vollständig mit Knochenzementteig gefüllt sind.

Ein weiterer Aspekt der Erfindung betrifft einen Träger, auf dem mehrere der oben beschriebenen Vorrichtungen zur Herstellung eines Spacers auf einer planen Fläche angeordnet sind. Jede der Vorrichtungen kann von Ausnehmungen umgeben sein, die es ermöglichen, die Vorrichtung durch Durchtrennen der zwischen den Ausnehmungen angeordneten Zwischenbereiche aus dem Träger zu entnehmen. Der Träger kann unterschiedliche Vorrichtungen umfassen, welche sich beispielsweise in der Form und/oder dem Volumen der ersten und/oder zweite Formnester unterscheiden. Beispielsweise kann dies dem Anwender ermöglichen, für unterschiedliche Patienten bzw. zu behandelnden Gelenke die jeweils geeignetste Vorrichtung auszuwählen. In Form eines solchen Trägers können daher erfindungsgemäße Vorrichtungen bereitgestellt werden, die für eine große Vielzahl unterschiedlicher Patienten und Einsatzzwecke verwendet werden können. Die unterschiedlichen Vorrichtungen auf einem Träger können sich beispielsweise auch durch das Vorhandensein oder der Ausgestaltung von den hierin beschriebenen Verbindungselementen, Stegen, Rillen, und/oder Befestigungselementen unterscheiden.

Zur weiteren Veranschaulichung der Erfindung ist auch ein Fingergelenkspacer beschrieben, welcher jedoch nicht Gegenstand der vorliegenden Erfindung ist. Ein solcher Fingergelenkspacer ist bevorzugt mithilfe einer hierin beschriebenen Vorrichtung herstellbar. Der Spacer kann einen Spacerkörper und einen Schaft umfassen. Der Schaft kann zur Verankerung in einem Knochengewebe eingerichtet sein. Der Spacerkörper kann eine artikulierende Fläche umfassen. Die artikulierende Fläche kann geeignet sein mithilfe einer weiteren artikulierenden Fläche eines korrespondierenden Spacerkörpers gemeinsam ein Gelenk zu bilden, wie hierin im Zusammenhang mit der beschriebenen Vorrichtung bereits ausführlich erläutert.

Weiterhin beschrieben sind zwei Spacer, deren Spacerkörper jeweils eine artikulierende Fläche aufweisen, die dazu eingerichtet sind, miteinander ein Gelenk zu bilden.

In einer Ausführungsform umfasst der Spacer Knochenzement, oder besteht daraus. Bevorzugt umfasst der Knochenzement PMMA. Der Knochenzement kann ein Antibiotikum umfassen. Beispiele für geeignete Antibiotika umfassen Gentamycin, Clindamycin und Vancomycin. Der Spacer ist bevorzugt eingerichtet, im Zusammenhang mit einem chirurgischen Eingriff ein natürliches Gelenk, beispielsweise ein Fingergelenk, eines Patienten temporär zu ersetzen. Mit "Fingergelenk" sind hierin alle Gelenke innerhalb der Finger, einschließlich des Daumens, bezeichnet. In einer Ausführungsform kann der Spacer im Bereich der Hand, beispielsweise im Bereich der Mittelhandknochen, eingesetzt werden.

Der Spacer, bevorzugt Fingergelenkspacer, kann ein artikulierender oder ein nicht artikulierender Spacer sein, wie hierin im Zusammenhang mit den erfindungsgemäßen Vorrichtungen auch andernorts beschrieben. Gegebenenfalls können die hierin beschriebenen artikulierenden Spacer paarweise miteinander verbunden werden, beispielsweise mithilfe von Knochenzement, um einen nicht artikulierenden Spacer zu bilden.

Ein weiterer Aspekt der Erfindung gemäß Anspruch 14 betrifft ein Verfahren zur Herstellung eines Fingergelenkspacers, umfassend die nachfolgenden Schritte,
(i) Bereitstellen einer hierin beschriebenen Vorrichtung zur Herstellung eines Spacers,
(ii) Inkontaktbringen des ersten Formnest mit dem zweiten Formnest unter Ausbildung eines nach außen abgedichteten Hohlraums,
   (ii.a) gegebenenfalls Verbinden des Unterteils mit dem Oberteil mit Hilfe eines ersten Befestigungselements, und eines dazu komplementären zweiten Befestigungselements,
   (ii.b.) gegebenenfalls, alternativ oder zusätzlich zu Schritt (ii.b.), Verbinden des Unterteils mit dem Oberteil durch Eingreifen eines Stegs in eine Rille,
(iii) Einbringen eines Knochenzementteigs in das erstes Formnest und das zweites Formnest,
(iv) Aushärten des Knochenzementteigs,
(v) Öffnen des Hohlraums durch zumindest teilweises Lösen des Unterteils von dem Oberteil, und
(vi) dadurch Erhalten eines Fingergelenkspacers aus Knochenzement, wobei Schritt (iii) zeitlich vor oder nach den Schritten (ii), (ii.a) und (ii.b) erfolgt.

In einem ersten Schritt (i) des Verfahrens wird eine erfindungsgemäße Vorrichtung zur Herstellung eines Fingergelenkspacers bereitgestellt. Hierzu wird eine Vorrichtung wie zuvor beschrieben verwendet, welche ein Unterteil und ein Oberteil umfasst, wobei das Unterteil mit dem Oberteil verbindbar ist, und wobei das Unterteil ein erstes Formnest umfasst, und das Oberteil ein zweites Formnest umfasst, wobei das erste Formnest eingerichtet ist, mit dem zweiten Formnest gemeinsam einen Hohlraum zu bilden, wobei das erste Formnest eine Spacerkörperform zum Formen eines Spacerkörpers umfasst, und das zweite Formnest eine Schaftform zum Formen eines Schafts umfasst.

In einem zweiten Schritt (ii) des Verfahrens wird das Unterteil der Vorrichtung mit dem Oberteil der Vorrichtung in Kontakt gebracht, sodass sich ein nach außen abgedichteter Hohlraum bildet, wie hierin im Zusammenhang mit der Vorrichtung zuvor ausführlicher beschrieben.

In einem optionalen weiteren Schritt (ii.a) des Verfahrens kann das Oberteil mit dem Unterteil mithilfe eines ersten Befestigungselements und eines dazu komplementären zweiten Befestigungselements verbunden werden. Beispielsweise kann das Oberteil einen Stift umfassen, der in eine dazu passende Aussparung auf dem Unterteil eingreifen kann.

In einem optionalen weiteren Schritt (ii.b) des Verfahrens kann das Unterteil mit dem Oberteil verbunden werden, indem ein Steg des Oberteils in eine Rille des Unterteils eingreift. Alternativ oder zusätzlich kann ein Steg des Unterteils in eine Rille des Oberteils eingreifen.

In einem dritten Schritt (iii) des Verfahrens wird Knochenzementteig in ein erstes und ein zweites Formnest der Vorrichtung eingebracht. Hierbei sollten die Formnester bevorzugt luftblasenfrei und vollständig mit dem Knochenzementteig gefüllt werden. Der Knochenzementteig kann entweder vor dem Schritt (ii), d. h. dem Inkontaktbringen des ersten Formnest mit dem zweiten Formnest, erfolgen, oder danach. Der Knochenzementteig kann gemäß einer Ausführungsform unmittelbar in die voneinander getrennt vorliegenden Formnester gegeben werden, beispielsweise durch Verstreichen des Knochenzementteigs mithilfe eines Spatels, oder mithilfe einer geeigneten Austragsvorrichtung. Falls die Formnester bereits aneinandergefügt sind, kann der Knochenzementteig durch eine Öffnung in einem der Formnester in den Hohlraum, der durch das erste und das zweite Formnest gemeinsam gebildet wird, eingespritzt werden.

In einem vierten Schritt (iv) das Verfahrens wird der Knochenzementteig ausgehärtet. Der Knochenzementteig enthält typischerweise reaktive Bestandteile, die sich nach dem Anmischen des Knochenzementteigs innerhalb weniger Minuten durch chemische Reaktion miteinander verbinden, sodass ein hartes, nicht mehr verformbares Material gebildet wird. Dieses Material wird als (ausgehärteter) Knochenzement bezeichnet, und bildet den erfindungsgemäßen Fingergelenkspacer.

In einem fünften Schritt (v) wird der Hohlraum, in dem sich der ausgehärtete Knochenzement befindet, geöffnet. Hierzu wird das Oberteil wieder von dem Unterteil getrennt, wobei das Oberteil gegebenenfalls noch über ein Verbindungselement mit dem Unterteil verbunden sein kann, jedoch die Formnester nicht mehr aneinander liegen.

In einem sechsten Schritt (vi) wird hierdurch ein Fingergelenkspacer aus Knochenzement erhalten. Dieser kann nun aus der Vorrichtung entnommen werden.

Die oben dargestellten Schritte des Verfahrens können grundsätzlich in jeder Reihenfolge erfolgen. In einer Ausführungsform erfolgt die Durchführung des Verfahrens in der oben dargestellten Reihenfolge. In einer anderen Ausführungsform erfolgt das Einbringen eines Knochenzementteigs beispielsweise vor dem Inkontaktbringen des ersten Formnests mit dem zweiten Formnest.

### BEISPIELE

Die Erfindung ist durch die Ansprüche definiert und wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

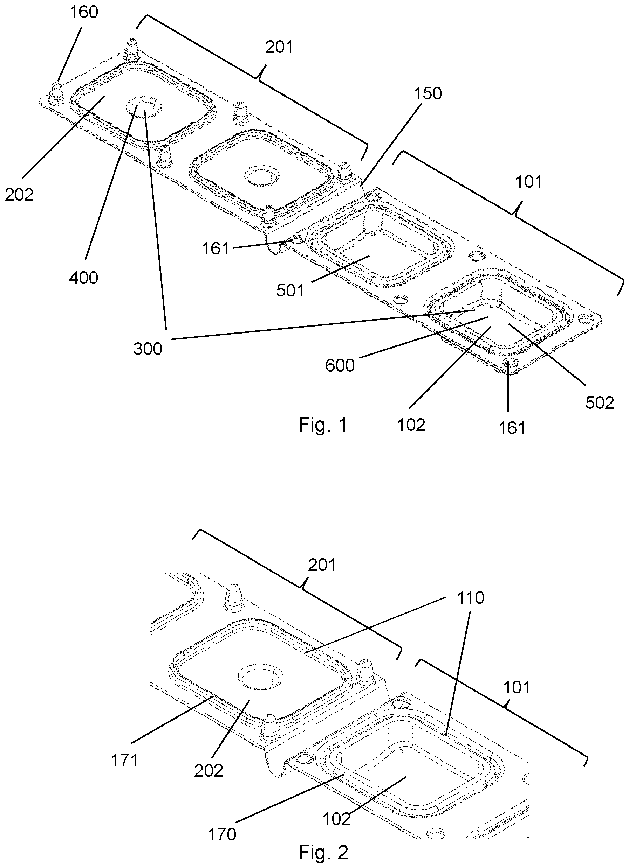

Figur 1 zeigt beispielhaft eine erste Ausführungsform der Erfindung. Die erfindungsgemäße Vorrichtung umfasst ein Unterteil **101,** welches mindestens ein erstes Formnest **102** umfasst. Im in Figur **1** dargestellten Beispiel umfasst das Unterteil **101** zwei erste Formnester **102.** Die ersten Formnester **102** umfassen jeweils eine Spacerkörperform **600** mit einer Stirnfläche **501, 502.** Im hier dargestellten Beispiel umfasst eines der beiden ersten Formnester **102** eine nach außen gewölbte, d. h. konvexe, Stirnfläche **501,** und das andere der beiden ersten Formnester **102** umfasst eine nach innen gewölbte, d. h. konkave, Stirnfläche **502.** Die mithilfe dieser Spacerkörperformen herstellbaren Spacerkörper erhalten dadurch zueinander passende artikulierende Flächen, die miteinander ein Gelenk bilden können. Das Unterteil umfasst weiterhin kreisförmige Aussparungen als erstes Befestigungselement **160.**

Die Vorrichtung umfasst weiterhin ein Oberteil 201 mit mindestens einem zweiten Formnest **202.** Im hier gezeigten Beispiel enthält das Oberteil **201** zwei zweite Formnester **202.** Das zweite Formnest **202** enthält jeweils eine Schaftform **400,** mit dessen Hilfe der Schaft eines Fingergelenkspacers hergestellt werden kann. Ein Teil der Schaftform **400** ist daher in diesem Beispiel in Form eines Kegelstumpfs ausgebildet. Die daraus herstellbaren Schäfte können daher eingerichtet sein, in ein Knochengewebe einzugreifen. Das Oberteil umfasst weiterhin Stiftförmige Vorsprünge als zweites Befestigungselement **161,** welche in die Aussparungen des Unterteils **160** eingreifen können, um die Formnester des Unterteils mit den Formnestern des Oberteils bündig aneinander auszurichten, und in dieser ausgerichteten Position zu halten. Hierbei wird durch ein erstes Formnest **102** und ein zweites Formnest **202** jeweils paarweise einen Hohlraum **300** gebildet. Dieser Hohlraum **300** ist eingerichtet, mit Knochenzementteig gefüllt zu werden, um einen Fingergelenkspacer aus dem Knochenzementteig zu formen. Weiterhin ist das Unterteil **101** mithilfe eines Verbindungselements **150** in Form eines Folienscharniers mit dem Oberteil **201** verbunden. Figur 2 zeigt eine vergrößerte Ansicht der in Figur 1 dargestellten Ausführungsform. Das Unterteil **101** umfasst eine Rille **170,** die entlang des Rands **110** eines ersten Formnests **102** angeordnet ist. Das Oberteil **201** umfasst einen Steg **171,** der entlang des Randes **110** eines zweiten Formnest **202** angeordnet ist.

Der Rand **110** bezeichnet jeweils die umlaufende Kante eines Formnests, bis zu der das betreffende Formnest mindestens mit Knochenzementteig zu befüllen ist, damit der Hohlraum **300** nach Zusammenfügen des Unterteils mit dem Oberteil vollständig mit Knochenzementteig gefüllt wird.

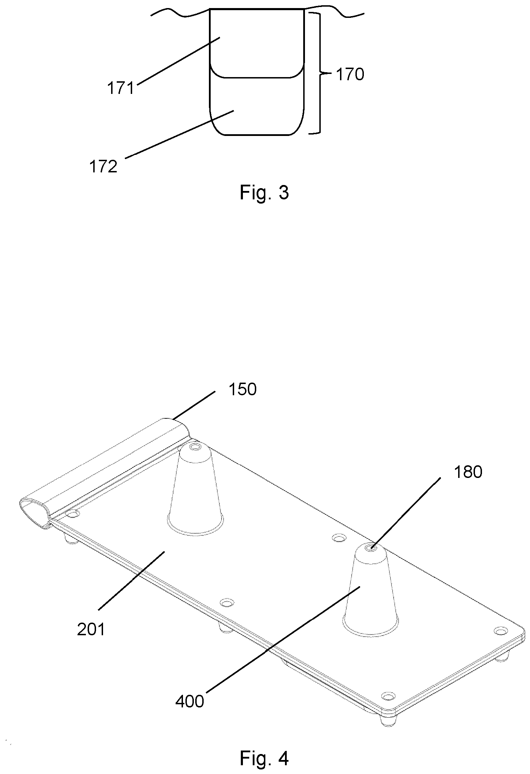

Figur 3 zeigt schematisch einen Ausschnitt einer Ausführungsform gemäß Figur 2, wobei das Unterteil wie oben beschrieben mit dem Oberteil verbunden ist, sodass jeweils ein erstes Formnest mit einem zweiten Formnest paarweise einen Hohlraum bildet. Hierbei greift ein Steg **171,** der entlang eines zweiten Formnest angeordnet ist, in eine Rille **170** eines ersten Formnests ein, wobei der Steg **171** die Rille **170** nach außen abdichtet, und gleichzeitig ein abgeschlossener Leerraum **172** zwischen dem Steg **171** und der Rille **170** gebildet wird. Der Leerraum **172** kann überschüssigen Knochenzementteig aufnehmen, der beim Zusammenfügen des Unterteils mit dem Oberteil aus den Formnestern nach außen gedrückt wird. Hierdurch kann die Bildung von Graten auf den abgeformten Fingergelenkspacern verringert oder vermindert werden.

Figur 4 zeigt eine Ausführungsform der erfindungsgemäßen Vorrichtung, bei der das Unterteil **101** mit dem Oberteil **201** unter Ausbildung eines nach außen abgedichteten Hohlraums in Kontakt gebracht sind. Das Unterteil **101** ist mit dem Oberteil **201** über ein flexibles Verbindungselement **150** verbunden. In der hier gezeigten Ausführungsform weist die Schaftform **400** eines zweiten Formnests **202** eine Öffnung **180** auf, welche an der Spitze der kegelstumpfförmigen Schaftform **400** angeordnet ist. Diese Öffnung **180** kann dazu dienen, Luft aus der Schaftform **400** des zweiten Formnests **202** entweichen zu lassen, wenn das zweite Formnest **202** mit Knochenzementteig befüllt wird. Dadurch kann eine vollständige, luftblasenfreie Befüllung der Vorrichtung, insbesondere des zweiten Formnests **202** und des Hohlraums **300** (in dieser Figur nicht zu sehen) besser gewährleistet werden. Gegebenenfalls kann die Öffnung **180** auch zum Einfüllen von Knochenzementteig genutzt werden.

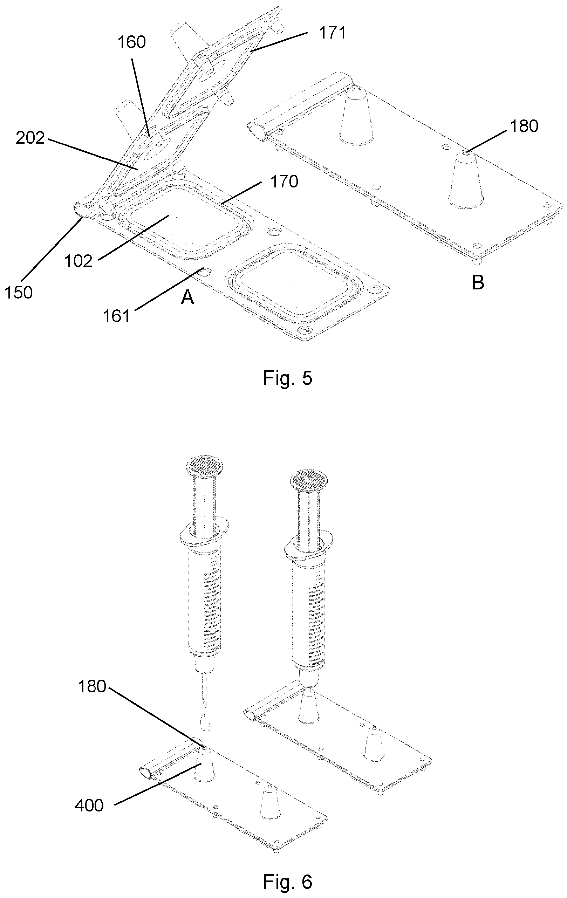

Figur 5 A zeigt eine Ausführungsform der erfindungsgemäßen Vorrichtung, bei der das Oberteil **201** unter Biegung des Verbindungselements **150** in Richtung des Unterteils **101** bewegt wird, um das erste Formnest **102** mit dem zweiten Formnests **202** in Kontakt zu bringen. In diesem Zustand können das erste Formnest **102** und das zweite Formnest **202** bereits mit Knochenzementteig befüllt sein.

Figur 5 B zeigt dieselbe Vorrichtung in einem verschlossenen bzw. "zusammengeklapptem" Zustand, bei welchem das erste Formnest **102** mit dem zweiten Formnest **202** in Kontakt gebracht ist, wobei die beiden Formnester gemeinsam einen Hohlraum ausbilden, der nach außen abgedichtet ist. Lediglich über eine optionale Öffnung **180** ist der Hohlraum nach außen zugänglich. Es können auch mehrere solcher Öffnungen vorgesehen sein. Das erste Befestigungselement **160** greift in das zweite Befestigungselement **161** ein, um die beiden Befestigungselemente fest miteinander zu verbinden, und das erste Formnest **102** gegenüber dem zweiten Formnests **202** in einer festen Position zu halten, sodass ein Fingergelenkspacer in der

Vorrichtung hergestellt werden kann.

Figur 6 zeigt eine Möglichkeit, die erfindungsgemäße Vorrichtung in einem geschlossenen Zustand gemäß Figur 5 B mit Knochenzementteig zu befüllen. Hierzu wird über eine Öffnung **180** Knochenzementteig ein Formnest eingebracht. Im hier gezeigten Beispiel ist die Öffnung **180** an der Spitze einer Schaftform **400** angebracht. Diese Art der Befüllung ist besonders bei der Verwendung von niedrigviskosem Knochenzementteigen vorteilhaft.

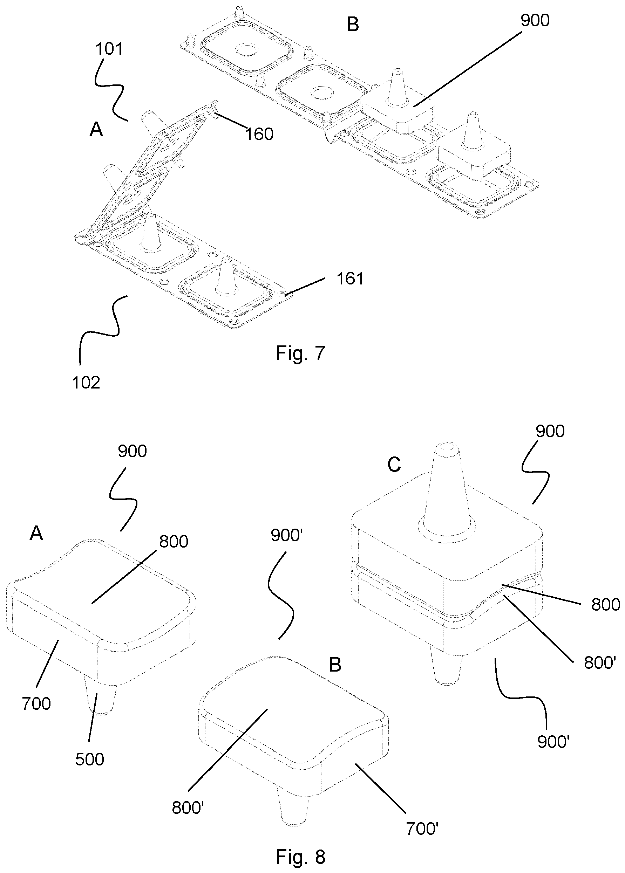

Figur 7 A zeigt eine erfindungsgemäße Vorrichtung, welche nach Aushärten des zuvor eingefügten Knochenzementteigs wieder geöffnet worden ist. Hierzu wird das erste Befestigungselement **160** vom zweiten Befestigungselement **161** gelöst, die Rille **170** aus dem Steg **171** bewegt, und das Oberteil **201** vom Unterteil **101** durch eine Rotationsbewegung gelöst, wobei das Unterteil **101** weiterhin über ein Verbindungselement **150** mit dem Oberteil **201** verbunden ist. Hierdurch wird die Vorrichtung geöffnet und der abgeformte Fingergelenkspacer **900** freigelegt.

Figur 7 B zeigt die Entnahme eines Fingergelenkspacers **900** aus Knochenzement aus der erfindungsgemäßen Vorrichtung, nachdem diese vollständig geöffnet wurde. In dieser hier gezeigten geöffneten Konfiguration liegen das Unterteil **101** und das Oberteil **201** in einer gemeinsamen Ebene.

Figur 8 A zeigt einen Fingergelenk-Spacer **900,** welcher einen Schaft **500** und einen Spacerkörper **700** umfasst. Der Spacerkörper **700** umfasst eine artikulierende Fläche **800,** welche eine abgerundete, im wesentlichen rechteckige Geometrie aufweist, und eine Wölbung in Richtung der Innenseite des Spacerkörpers **700** aufweist. Die artikulierende Fläche **800** ist daher konkav.

Figur 8 B zeigt einen Fingergelenkspacer 900', welcher eine zu dem in Figur 8 A gezeigten Fingergelenkspacer 900 komplementäre Form aufweist. Dies bedeutet, dass der Fingergelenkspacer 900' eine artikulierende Fläche 800' umfasst, welche eingerichtet ist, durch Zusammenwirkung mit der artikulierenden Fläche 800 gemeinsam ein Gelenk aus den beiden Fingergelenkspacern zu bilden. Dazu weist der Fingergelenkspacer 900' einen Spacerkörper 700' auf, welcher eine konvexe, d. h. nach außen gewölbte, artikulierende Fläche 800' umfasst. Figur 8 C zeigt, wie der Fingergelenkspacer 900 gemäß Figur 8 A und der Fingergelenkspacer 900' gemäß der Figur 8 B gemeinsam miteinander ein Gelenk bilden, indem ihre beiden artikulierenden Flächen **800** und 800' miteinander in Kontakt gebracht werden.

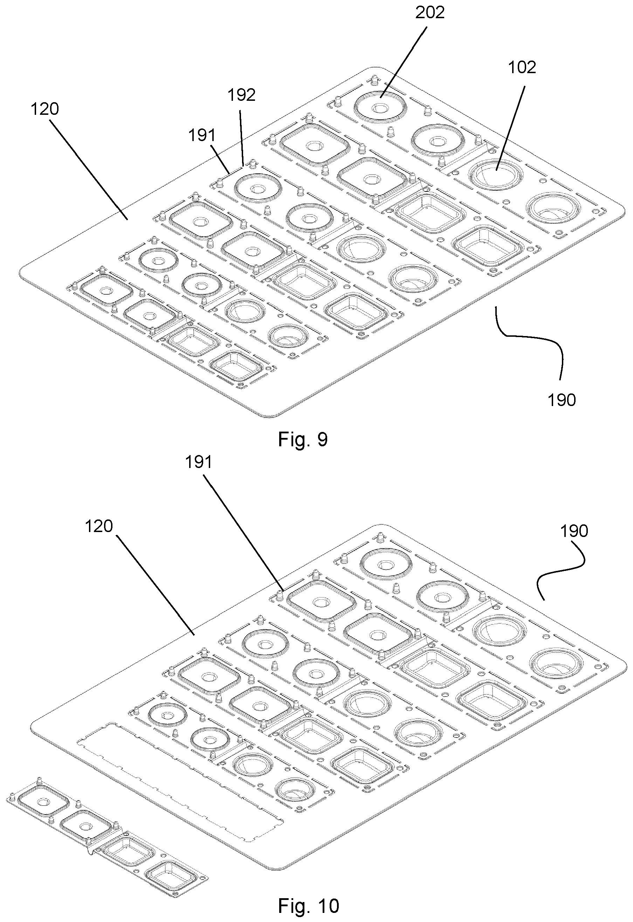

Figur 9 zeigt einen Träger 190, auf dem verschiedene Vorrichtungen gemäß den vorangehenden Figuren auf einer planen Fläche **120** angeordnet sind. Die einzelnen Vorrichtungen sind von mehreren Ausnehmungen **191** umgeben, welche ein Heraustrennen der Vorrichtungen ermöglichen, indem die Zwischenbereiche **192,** welche zwischen den Ausnehmungen angeordnet sind, durchtrennt werden, zum Beispiel mit einer Schere oder einem anderen Schneidwerkzeug. Auf einem Träger können unterschiedliche erfindungsgemäße Vorrichtungen angeordnet sein, beispielsweise Vorrichtungen mit Formnestern **102, 202,** die unterschiedliche Geometrien und/oder Größen aufweisen. Beispielsweise können Formnester mit unterschiedlichen Volumen und/oder Formnester mit im Wesentlichen quaderförmiger und halbkugelförmige Form auf einem gemeinsamen Träger **190** angeordnet sein.

Figur 10 zeigt einen Träger **190** gemäß Figur 9, bei dem eine der erfindungsgemäßen Vorrichtungen durch Auftrennen der Zwischenbereiche aus dem Träger **190** herausgelöst ist.

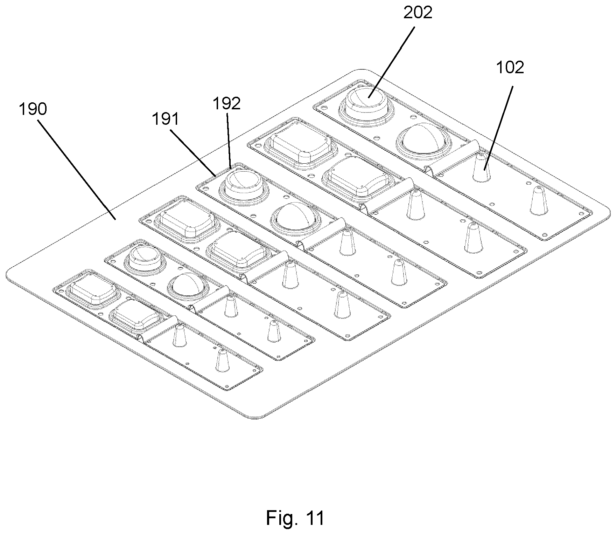

Figur 11 zeigt einen Träger **190** gemäß Figur 9 aus einer anderen Ansichtsperspektive. Die Geometrie der verschiedenen geformten Formnester **102, 202** lässt sich aus dieser Ansicht besonders gut erkennen.

### LISTE DER EZUGSZEICHEN

- 101: Unterteil
- 102: erstes Formnest
- 110: Rand
- 120: plane Fläche
- 150: Verbindungselement
- 160: erstes Befestigungselement
- 161: zweites Befestigungselement
- 170: Rille
- 171: Steg
- 172: Leerraum
- 180: Öffnung
- 190: Träger
- 191: Ausnehmung
- 192: Zwischenbereich
- 201: Oberteil
- 202: zweites Formnest
- 300: Hohlraum
- 400: Schaftform
- 500: Schaft
- 501, 502: Stirnfläche
- 600: Spacerkörperform
- 700, 701': Spacerkörper
- 800, 800': artikulierende Fläche
- 900, 900': Fingergelenkspacer

## Patentansprüche

1. Vorrichtung zur Herstellung von Fingergelenkspacern (900, 900') umfassend ein Unterteil (101) und ein Oberteil (201), wobei das Unterteil (101) mit dem Oberteil (201) verbindbar ist, wobei das Unterteil (101) ein erstes Formnest (102) umfasst, und das Oberteil (201) ein zweites Formnest (202) umfasst, wobei das erste Formnest (102) eingerichtet ist, mit dem zweiten Formnest (202) gemeinsam einen Hohlraum (300) zu bilden, wobei das erste Formnest (102) eine Spacerkörperform (600) zum Formen eines Spacerkörpers (700) umfasst, und das zweite Formnest (202) eine Schaftform (400) zum Formen eines Schafts (500) umfasst, **dadurch gekennzeichnet, dass** die Vorrichtung ein biegsames Verbindungselement (150) in Form eines Folienscharniers umfasst, welches das Unterteil (101) mit dem Oberteil (201) verbindet.

2. Vorrichtung nach Anspruch 1, wobei der Hohlraum (300) ein Volumen von 0,1 bis 5 cm³, bevorzugt 0,3 bis 4,0 cm³, umfasst.

3. Vorrichtung nach Anspruch 1 oder 2, wobei die Schaftform (400) die Form eines Rotationskörpers, einer Pyramide oder eines Pyramidenstumpfs umfasst.

4. Vorrichtung nach einem der vorangehenden Ansprüche, wobei die Spacerkörperform (600) im Wesentlichen quaderförmig oder im Wesentlichen halbkugelförmig ausgebildet ist.

5. Vorrichtung nach einem der vorangehenden Ansprüche, wobei die Spacerkörperform (600) eine Stirnfläche (501, 502) umfasst, welche eingerichtet ist, eine artikulierende Fläche (800) zu formen, die geeignet ist, mit einer weiteren artikulierenden Fläche (800') eines korrespondierenden Spacerkörpers (700') gemeinsam ein Gelenk zu bilden.

6. Vorrichtung nach Anspruch 5, wobei die Stirnfläche (501, 502) eine gewölbte Form aufweist, die bevorzugt konvex oder konkav ist.

7. Vorrichtung nach einem der vorangehenden Ansprüche, wobei das Verbindungselement bevorzugt jeweils randständig an dem Unterteil (101) und dem Oberteil (201) angeordnet ist.

8. Vorrichtung nach einem der vorangehenden Ansprüche, weiterhin umfassend ein erstes Befestigungselement (160), und ein dazu komplementäres zweites Befestigungselement (161), wobei die Vorrichtung eingerichtet ist, das Unterteil (101) durch Eingreifen des ersten Befestigungselements (160) in das zweite Befestigungselement (161) mit dem Oberteil (201) zu verbinden.

9. Vorrichtung nach einem der vorangehenden Ansprüche, weiterhin umfassend eine Rille (170), die umlaufend entlang mindestens eines der Formnester (102, 202) angeordnet ist.

10. Vorrichtung nach Anspruch 9, weiterhin umfassend einen Steg (171), der angeordnet und eingerichtet ist, formschlüssig in die Rille (170) einzugreifen, um durch Verbindung des Unterteils (101) mit dem Oberteil (201) den Hohlraum (300) nach außen abzudichten.

11. Vorrichtung nach Anspruch 10, wobei die Rille (170) und der Steg (171) angeordnet und eingerichtet sind, um durch Eingreifen des Stegs (171) in die Rille (170) einen Leerraum (172) zwischen dem Steg und der Rille auszubilden, der zur Aufnahme überschüssigen Knochenzements eingerichtet ist.

12. Vorrichtung nach einem der vorangehenden Ansprüche, wobei das Unterteil (101) und/oder das Oberteil (201) eine Öffnung (180) aufweist, die zum Entlüften des Hohlraums (300) eingerichtet ist.

13. Vorrichtung nach einem der vorangehenden Ansprüche, wobei das Unterteil (101) und/oder das Oberteil (201) ein Polymer umfassen, das ausgewählt ist aus der Gruppe bestehend aus Kautschuk, Silikonkautschuk, Synthesekautschuk, Ethylen-Propylen-DienKautschuk (EPDM), Polyethylen, Polyetheretherketon, und Polypropylen, bevorzugt Polypropylen, wobei das Polymer weiter bevorzugt transparent oder transluzent ist.

14. Verfahren zur Herstellung eines Fingergelenkspacers, umfassend die nachfolgenden Schritte,
(i) Bereitstellen einer Vorrichtung nach einem der Ansprüche 1 bis 13,
(ii) Inkontaktbringen des ersten Formnest (102) mit dem zweiten Formnest (202) der Vorrichtung unter Ausbildung eines nach außen abgedichteten Hohlraums (300),
(ii.a) gegebenenfalls Verbinden des Unterteils (101) mit dem Oberteil (201) mit Hilfe eines ersten Befestigungselements (160), und eines dazu komplementären zweiten Befestigungselements (161),
(ii.b.) gegebenenfalls, alternativ oder zusätzlich zu Schritt (ii.b.), Verbinden des Unterteils (101) mit dem Oberteil (201) durch Eingreifen eines Stegs (171) in eine Rille (170),
(iii) Einbringen eines Knochenzementteigs in das erste Formnest (102) und das zweite Formnest (202),
(iv) Aushärten des Knochenzementteigs,
(v) Öffnen des Hohlraums (300) durch zumindest teilweises Lösen des Unterteils (101) von dem Oberteil (201), und
(vi) dadurch Erhalten eines Fingergelenkspacers aus Knochenzement, wobei Schritt (iii) zeitlich vor oder nach den Schritten (ii), (ii.a) und (ii.b) erfolgt.

## Claims

1. A device for producing finger joint spacers (900, 900'), comprising a lower part (101) and an upper part (201), it being possible for the lower part (101) to be connected to the upper part (201), the lower part (101) comprising a first mold cavity (102), and the upper part (201) comprising a second mold cavity (202), the first mold cavity (102) being configured to form a cavity (300) together with the second mold cavity (202), the first mold cavity (102) comprising a spacer body mold (600) for forming a spacer body (700), and the second mold cavity (202) comprising a shaft mold (400) for forming a shaft (500), **characterized in that** the device comprises a flexible connecting element (150) in the form of a film hinge, which connects the lower part (101) to the upper part (201).

2. The device according to claim 1, wherein the cavity (300) has a volume of 0.1 to 5 cm³, preferably 0.3 to 4.0 cm³.

3. The device according to claim 1 or 2, wherein the shaft mold (400) is in the shape of a solid of revolution, a pyramid or a truncated pyramid.

4. The device according to any of the preceding claims, wherein the spacer body mold (600) is substantially cuboidal or substantially hemispherical.

5. The device according to any of the preceding claims, wherein the spacer body mold (600) comprises an end face (501, 502) which is configured to form an articulating surface (800) which is suitable for jointly forming a joint with a further articulating surface (800') of a corresponding spacer body (700').

6. The device according to claim 5, wherein the end face (501, 502) has a curved shape, which is preferably convex or concave.

7. The device according to any of the preceding claims, wherein the connecting element is preferably arranged at the edge of the lower part (101) and the upper part (201).

8. The device according to any of the preceding claims, further comprising a first fastening element (160) and a second fastening element (161) complementary thereto, wherein the device is configured to connect the lower part (101) to the upper part (201) by engagement of the first fastening element (160) in the second fastening element (161).

9. The device according to any of the preceding claims, further comprising a groove (170) arranged peripherally along at least one of the mold cavities (102, 202).

10. The device according to claim 9, further comprising a projection (171) which is arranged and configured to engage form-fittingly in the groove (170) in order to outwardly seal the cavity (300) by connecting the lower part (101) to the upper part (201).

11. The device according to claim 10, wherein the groove (170) and the projection (171) are arranged and configured to form a void (172) between the projection and the groove by engagement of the projection (171) in the groove (170), which void is configured to receive excess bone cement.

12. The device according to any of the preceding claims, wherein the lower part (101) and/or the upper part (201) comprises an opening (180) which is configured to vent the cavity (300).

13. The device according to any of the preceding claims, wherein the lower part (101) and/or the upper part (201) comprise a polymer selected from the group consisting of rubber, silicone rubber, synthetic rubber, ethylene-propylene-diene rubber (EPDM), polyethylene, polyetheretherketone, and polypropylene, preferably polypropylene, wherein the polymer is further preferably transparent or translucent.

14. A method for producing a finger joint spacer, which comprises the following steps
(i) providing a device according to any of claims 1 to 13,
(ii) bringing the first mold cavity (102) into contact with the second mold cavity (202) of the device to form an outwardly sealed cavity (300),
(ii.a) optionally connecting the lower part (101) to the upper part (201) by means of a first fastening element (160) and a second fastening element (161) complementary thereto,
(ii.b.) optionally, alternatively or in addition to step (ii.b.), connecting the lower part (101) to the upper part (201) by engagement of a projection (171) in a groove (170),
(iii) introducing a bone cement paste into the first mold cavity (102) and the second mold cavity (202),
(iv) curing the bone cement paste,
(v) opening the cavity (300) by at least partially detaching the lower part (101) from the upper part (201), and
(vi) thereby obtaining a finger joint spacer made of bone cement, wherein step (iii) takes place before or after steps (ii), (ii.a) and (ii.b).

## Revendications

1. Dispositif permettant la fabrication d'écarteurs d'articulation de doigt (900, 900'), comprenant une partie inférieure (101) et une partie supérieure (201), dans lequel la partie inférieure (101) peut être reliée à la partie supérieure (201), dans lequel la partie inférieure (101) comprend une première cavité de moulage (102), et la partie supérieure (201) comprend une seconde cavité de moulage (202), dans lequel la première cavité de moulage (102) est conçue pour former un espace creux (300) conjointement avec la seconde cavité de moulage (202), dans lequel la première cavité de moulage (102) comprend un moule pour corps formant espaceur (600) pour le moulage d'un corps formant espaceur (700), et la seconde cavité de moulage (202) comprend un moule pour tige (400) pour le moulage d'une tige (500), **caractérisé en ce que** le dispositif comprend un élément de liaison (150) flexible sous la forme d'une charnière en feuille qui relie la partie inférieure (101) à la partie supérieure (201).

2. Dispositif selon la revendication 1, dans lequel l'espace creux (300) comprend un volume allant de 0,1 à 5 cm³, de préférence de 0,3 à 4,0 cm³.

3. Dispositif selon la revendication 1 ou 2, dans lequel le moule pour tige (400) comprend la forme d'un corps de rotation, d'une pyramide ou d'une pyramide tronquée.

4. Dispositif selon l'une des revendications précédentes, dans lequel le moule pour corps formant espaceur (600) est réalisé de manière sensiblement parallélépipédique ou sensiblement hémisphérique.

5. Dispositif selon l'une des revendications précédentes, dans lequel le moule pour corps formant espaceur (600) comprend une surface frontale (501, 502) conçue pour mouler une surface d'articulation (800) apte à former, conjointement avec une autre surface d'articulation (800') d'un corps formant espaceur (700') correspondant, une articulation.

6. Dispositif selon la revendication 5, dans lequel la surface frontale (501, 502) présente une forme bombée qui est de préférence convexe ou concave.

7. Dispositif selon l'une des revendications précédentes, dans lequel l'élément de liaison est de préférence disposé respectivement près du bord de la partie inférieure (101) et de la partie supérieure (201).

8. Dispositif selon l'une des revendications précédentes, comprenant en outre un premier élément de fixation (160) et un second élément de fixation (161) qui lui est complémentaire, dans lequel le dispositif est conçu pour relier la partie inférieure (101) à la partie supérieure (201) en faisant venir en prise le premier élément de fixation (160) dans le second élément de fixation (161).

9. Dispositif selon l'une des revendications précédentes, comprenant en outre une rainure (170) disposée de manière circonférentielle le long d'au moins une des cavités de moulage (102, 202).

10. Dispositif selon la revendication 9, comprenant en outre une barrette (171) disposée et conçue pour venir en prise par complémentarité de forme dans la rainure (170) afin d'assurer l'étanchéité de l'espace creux (300) vis-à-vis de l'extérieur en reliant la partie inférieure (101) à la partie supérieure (201).

11. Dispositif selon la revendication 10, dans lequel la rainure (170) et la barrette (171) sont disposées et conçues pour réaliser un espace vide (172) entre la barrette et la rainure, lequel est conçu pour recevoir du ciment osseux excédentaire, en faisant venir en prise la barrette (171) dans la rainure (170).

12. Dispositif selon l'une des revendications précédentes, dans lequel la partie inférieure (101) et/ou la partie supérieure (201) présentent une ouverture (180) conçue pour évacuer l'air de l'espace creux (300).

13. Dispositif selon l'une des revendications précédentes, dans lequel la partie inférieure (101) et/ou la partie supérieure (201) comprennent un polymère choisi dans le groupe constitué de caoutchouc, caoutchouc de silicone, caoutchouc synthétique, caoutchouc éthylène-propylène-diène (EPDM), polyéthylène, polyétheréthercétone, et polypropylène, de préférence du polypropylène, dans lequel le polymère est en outre de préférence transparent ou translucide.

14. Procédé permettant la fabrication d'un écarteur d'articulation de doigt, comprenant les étapes suivantes,
(i) fourniture d'un dispositif selon l'une des revendications 1 à 13,
(ii) mise en contact de la première cavité de moulage (102) avec la seconde cavité de moulage (202) du dispositif en réalisant un espace creux (300) étanche vers l'extérieur,
(ii.a) éventuellement, liaison de la partie inférieure (101) à la partie supérieure (201) à l'aide d'un premier élément de fixation (160), et d'un second élément de fixation (161) qui lui est complémentaire,
(ii.b.) éventuellement, alternativement ou en plus de l'étape (ii.b.), liaison de la partie inférieure (101) à la partie supérieure (201) en faisant venir en prise une barrette (171) dans une rainure (170),
(iii) introduction d'une pâte de ciment osseux dans la première cavité de moulage (102) et dans la seconde cavité de moulage (202),
(iv) durcissement de la pâte de ciment osseux,
(v) ouverture de l'espace creux (300) en détachant au moins partiellement la partie inférieure (101) de la partie supérieure (201), et
(vi) obtention en conséquence d'un écarteur d'articulation de doigt en ciment osseux, dans lequel l'étape (iii) est effectuée chronologiquement avant ou après les étapes (ii), (ii.a) et (ii.b).
